# EUROPEAN PATENT SPECIFICATION

(11) **EP 1 539 269 B1**
(45) Date of publication and mention of the grant of the patent: **21.02.2007**
(21) Application number: 03754476.4
(22) Date of filing: 08.09.2003
(51) Int. Cl.: A61L 31/02, A61L 31/18

(54) **STENTS COMPRISING A MOLYBDENUM/RHENIUM ALLOY**
STENTS ENTHALTEND EINE MOLYBDÄN/RHENIUM-LEGIERUNG
STENTS COMPRENANT UN ALLIAGE AU MOLYBDENE-RHENIUM

(30) Priority: 09.09.2002 US 237517
(43) Date of publication of application: 15.06.2005
(73) Proprietor: Boston Scientific Limited, St. Michael, Barbados, West Indies (BB)
(72) Inventor: XU, Yixin, Mewton, MA 02462 (US)
(74) Representative: Altenburg, Udo
(86) International application number: PCT/US2003/028232
(87) International publication number: WO 2004/022122

(56) References cited:
- WO-A-95/30384

## Description

### TECHNICAL FIELD

The invention relates to stents.

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body Endoprostheses stents include covered stents, also sometimes called "stent-grafts".

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self-expand by its own internal elastic restoring force.

To support a passageway open, endoprostheses are sometimes made of relatively strong materials, such as stainless steel or Nitinol (a nickel-titanium alloy), formed into struts or wires. These materials, however, can be relatively radiolucent. That is, the materials may not be easily visible under X-ray fluoroscopy, which is a technique used to locate and to monitor the endoprostheses during and after delivery. To enhance their visibility (e.g., by increasing their radiopacity), the endoprostheses can be coated with a relatively radiopaque material, such as gold.

### SUMMARY

The invention features a stent including a molybdenum/rhenium alloy providing the stent with good radiopacity. In addition, molybdenum/rhenium alloys are strong, flexible and have good ductibility.

The molybdenum/rhenium alloy includes between 10% and 70% molybdenum by weight and between 35% and 55%, rhenium by weight. Preferred molybdenum/rhenium alloys have a density of from 8 and 19 g/cm³, more preferably between 10 g/cm³ and 15 g/cm³. The molybdenum/rhenium alloys preferably have a tensile strength of from 276 MPa (40 ksi) to 2068 MPa (300 ksi), more preferably between 896 MPa (130 ksi) and 1310 MPa (190 ksi), and a modulus of elasticity from 324054 MPa (47,000 ksi) to 461948 MPa (67,000 ksi).

Other aspects, features, and advantages of the invention will be apparent from the description of the embodiments thereof, and from the claims.

### DESCRIPTION OF DRAWINGS

FIG. 1 is a perspective view of a stent composed of a molybdenum/rhenium alloy;
FIG. 2 is a perspective view of a composite tubing including a molybdenum/rhenium alloy;
FIG. 3 is a perspective view, in close-up, of a stent partially composed of a molybdenum/rhenium alloy;
FIG. 4 is a schematic view, in close-up, of a stent partially composed of a molybdenum/rhenium alloy; and
FIG. 5 is a schematic view, in close-up, of a stent partially composed of a molybdenum/rhenium alloy.

### DETAILED DESCRIPTION

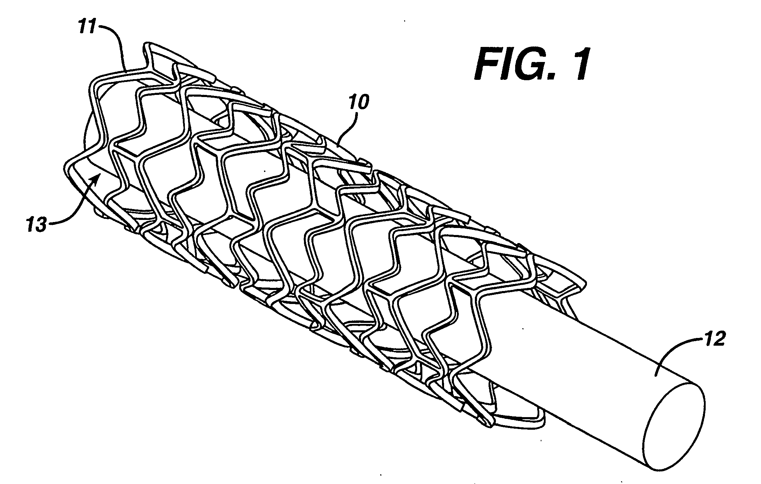

Referring to Fig. 1, a support 12 carries a stent 10, which is the form of a tubular member including struts 11 and openings 13. Depending on the type of stent, support 12 can be a balloon catheter or a catheter shaft.

The stent is composed of a molybdenum/rhenium alloy. The alloy may contain other metals in addition to molybdenum and rhenium, but preferably the alloy consists essentially of molybdenum and rhenium. Molybdenum/rhenium alloy tubing, sheet, foil, and wire is available from Rhenium Alloys, Inc., of 1329 Taylor Street, Elyria, Ohio. An example of a preferred tubing is composed of an alloy including 47.5% by weight rhenium and the balance molybdenum. This alloy has a density of 13.5 g/cm³, a modulus of elasticity of 369718 MPa (53,623 ksi), a tensile strength of 848 MPa (123 ksi), and a percent elongation of 22%.

The molybdenum/rhenium alloy may include, for example, between 40% and 50% rhenium by weight and, for example, between 40% and 70% or between 50% and 60% molybdenum by weight.

A sheet or foil can be folded and welded to provide a tube using inert gas or electron beam methods, with appropriate protection against oxidation. The tubing can then be drawn or extruded to the desired diameter, or used to fabricate a stent directly. Depending on the application, the stent may have a diameter of between, for example, 1 mm to 46 mm (1 mm to 5 mm for a coronary stent, 20 mm to 46 mm for AAA and TAA stents, in between for peripheral and non-vascular stents). The tubing alternatively can be made, for example, by seamless drawing or extrusion.

After the molybdenum/rhenium alloy tubing has been drawn to the desired diameter, portions of the tubing are removed to provide the strut 11/opening 13 arrangement. The portions can be removed by laser cutting, as described, for example, in U.S. Pat. 5,780,807. Alternatively, the portions can be removed by electrochemical machining, electrical discharge machining, abrasive cutting/grinding methods, or photoetching. Stent 10 can then be finished by electropolishing to a smooth finish, by conventional methods. The stent also can be annealed.

Stent 10 can then be delivered and expanded by generally conventional methods.

Stent 10 can be a part of a stent-graft. The stent-graft can be a stent attached to a biocompatible, non-porous or semi-porous polymer matrix made of polytetrafluoroethylene (PTFE), expanded PTFE, polyethylene, urethane, or polypropylene. Stent 10 can include a releasable therapeutic agent or a pharmaceutically active compound, such as described in U.S. Patent No. 5,674,242, and commonly-assigned U.S.S.N. 09/895,415, filed July 2, 2001. The therapeutic agents or pharmaceutically active compounds can include, for example, anti-thrombogenic agents, antioxidants, anti-inflammatory agents, anesthetic agents, anti-coagulants, and antibiotics.

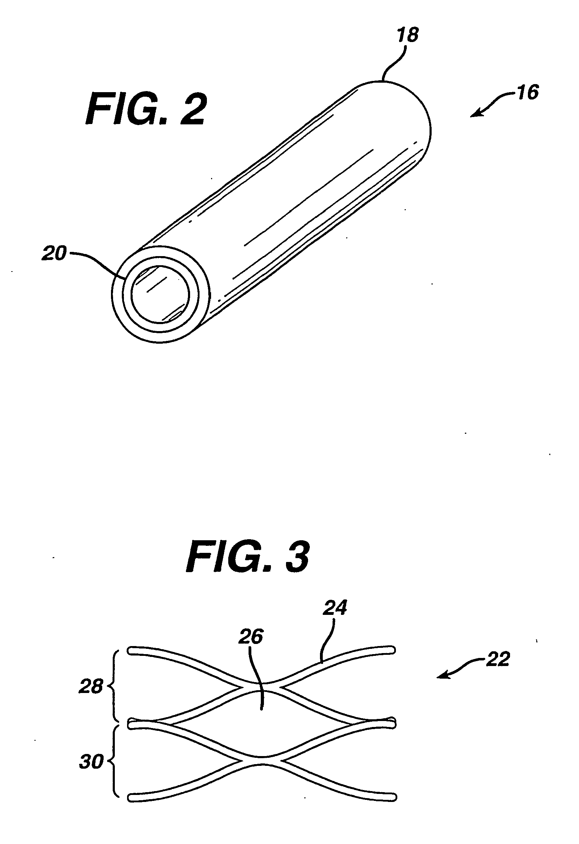

Referring to Fig. 2, a composite tubing 16 includes an outer layer 18 composed of nitinol and an inner layer 20 composed of the molybdenum/rhenium alloy. The composite tubing can be made by co-drawing of two components, by coating one material to another, using CVD (chemical vapor deposition) and PVD (physical vapor deposition) method, or by electric plating one material to another. Outer layer 18 alternatively can be composed of stainless steel. Tubing 16 can be drawn and converted into a stent (with stents) using the methods described above. The two-layer stent can have a combination of properties fully or in part provided by each layer. Preferred two-layer stents can have, for example, good radiopacity, flexibility, and strength.

The portion of outer layer 18 and inner layer 20 in tube 16 (and in the resulting stent) can be reversed.

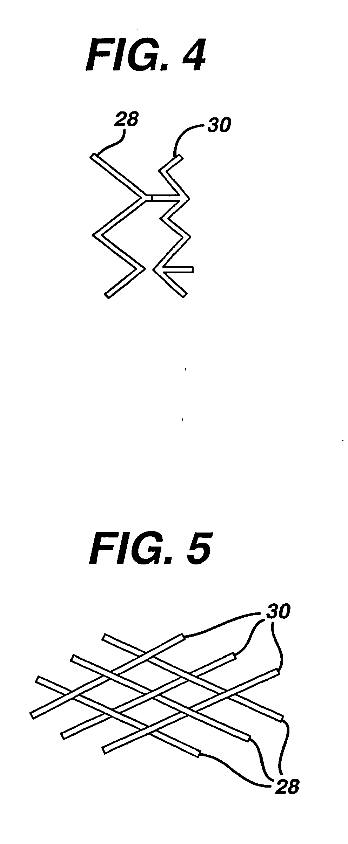

Referring to Fig. 3, an alternative embodiment of a stent 22 includes struts 24, openings 26, and portions 28 and 30. Portion 28 is composed of the molybdenum/rhenium alloy and portion 30 is composed of, for example, nitinol or stainless steel. Portion 28 may be in the form of a band or bands positioned periodically along the length of the stent. Alternatively, portion 28 may be combined with portion 30 as shown in Figs. 4 and 5. The stent may include, for example, from 10% to 90% or 100% of the molybdenum/rhenium alloy by weight. Molybdenum/rhenium alloy portion(s) 28 can be joined to portion 30 by, for example, welding. Portion 28 provides the stent with enhanced radiopacity. When portion 30 is composed of nitinol or other self-expanding material, the stent will have some portion(s) that are self-expanding and other portion(s) (the molybdenum/rhenium portion(s)) that are balloon expandable.

Other embodiments are within the claims.

## Claims

1. A stent (10) comprising a tubular member comprising a molybdenum/rhenium alloy, wherein the molybdenum/rhenium alloy includes between 10 % and 70 % molybdenum by weight and between 35 % and 55 % rhenium by weight.

2. The stent (10) of claim 1, wherein the molybdenum/rhenium alloy includes between 45 % and 65 % molybdenum by weight.

3. The stent (10) of claim 1, wherein the tubular member comprises a first portion (28) comprising the molybdenum/rhenium alloy and a second portion (30).

4. The stent (10) of claim 3, wherein the second portion (30) comprises a material selected from the group consisting of stainless steel and nickel-titanium alloy.

5. The stent (10) of claim 1, wherein the tubular member comprises a first layer (20) comprising the molybdenum/rhenium alloy and a second layer (18).

6. The stent (10) of claim 5, wherein the second layer (18) is outside the first layer (20).

7. The stent (10) of claim 1, wherein the tubular member comprises struts (11).

8. The stent (10) of claim 3, wherein the tubular member has struts (24) comprising the first portion (28) and struts (24) comprising the second portion (30).

9. The stent (10) of claim 1, wherein the molybdenum/rhenium alloy has a density of at least 8 g/cm³.

10. The stent (10) of claim 9, wherein the molybdenum/rhenium alloy has a density of less than 19 g/cm³.

11. The stent (10) of claim 1, wherein the molybdenum/rhenium alloy has a tensile strength of from about 276 MPa (40 ksi) to about 2068 MPa (300 ksi).

12. The stent (10) of claim 1, wherein the molybdenum/rhenium alloy has a modulus of elasticity of between about 324,054 (47,000) to about 461,949 MPa (67,000 ksi).

## Patentansprüche

1. Stent (10), welcher ein rohrförmiges Bauteil aufweist, welches eine Molybdän/Rhenium-Legierung aufweist, wobei die Molybdän/Rhenium-Legierung zwischen 10 und 70 Gew.-% Molybdän und zwischen 35 und 55 Gew.-% Rhenium enthält.

2. Stent (10) nach Anspruch 1, bei welchem die Molybdän/Rhenium-Legierung zwischen 45 und 65 Gew.-% Molybdän enthält.

3. Stent (10) nach Anspruch 1, bei welchem das rohrförmige Bauteil einen ersten Abschnitt (28), welcher die Molybdän/Rhenium-Legierung aufweist, und einen zweiten Abschnitt (30) aufweist.

4. Stent (10) nach Anspruch 3, bei welchem der zweite Abschnitt (30) ein Material aufweist, ausgewählt aus der Gruppe bestehend aus rostfreiem Stahl und Nickel-Titan-Legierung.

5. Stent (10) nach Anspruch 1, bei welchem das rohrförmige Bauteil eine erste Schicht (20), welche die Molybdän/Rhenium-Legierung aufweist, und eine zweite Schicht (18) aufweist.

6. Stent (10) nach Anspruch 5, bei welchem die zweite Schicht (18) außerhalb der ersten Schicht (20) ist.

7. Stent (10) nach Anspruch 1, bei welchem das rohrförmige Bauteil Verstrebungen (11) aufweist,

8. Stent (10) nach Anspruch 3, bei welchem das rohrförmige Bauteil Verstrebungen (24), welche den ersten Abschnitt (28) aufweisen, und Verstrebungen (24), welche den zweiten Abschnitt (30) aufweisen, hat.

9. Stent (10) nach Anspruch 1, bei welchem die Molybdän/Rhenium-Legierung einen Dichte von mindestens 8 g/cm³ hat.

10. Stent (10) nach Anspruch 9, bei welchem die Molybdän/Rhenium-Legierung eine Dichte von weniger als 19 g/cm³ hat.

11. Stent (10) nach Anspruch 1, bei welchem die Molybdän/Rhenium-Legierung eine Zugfestigkeit von ungefähr 276 MPa (40 ksi) bis ungefähr 2068 MPa (300 ksi) hat.

12. Stent (10) nach Anspruch 1, bei welchem die Molybdän/Rhenium-Legierung einen Elastizitätsmodul zwischen ungefähr 324,054 (47.000) bis ungefähr 461,949 MPa (67.000 ksi) hat.

## Revendications

1. Stent (10) comprenant un élément tubulaire comprenant un alliage de molybdène/rhénium, dans lequel l'alliage de molybdène/rhénium inclut entre 10 % et 70 % de molybdène en poids et entre 35 % et 55 % de rhénium en poids.

2. Stent (10) selon la revendication 1, dans lequel l'alliage de molybdène/rhénium inclut entre 45 % et 65 % de molybdène en poids.

3. Stent (10) selon la revendication 1, dans lequel l'élément tubulaire comprend une première partie (28) comportant l'alliage de molybdène/rhénium et une seconde partie (30).

4. Stent (10) selon la revendication 3, dans lequel la seconde partie (30) comprend un matériau sélectionné dans le groupe constitué par l'acier inoxydable et l'alliage de nickel et de titane.

5. Stent (10) selon la revendication 1, dans lequel l'élément tubulaire comprend une première couche (20) comprenant l'alliage de molybdène/rhénium et une seconde couche (18).

6. Stent (10) selon la revendication 5, dans lequel la seconde couche (18) est à l'extérieur de la première couche (20).

7. Stent (10) selon la revendication 1, dans lequel l'élément tubulaire comprend des montants (11).

8. Stent (10) selon la revendication 3, dans lequel l'élément tubulaire a des montants (24) comprenant la première partie (28) et des montants (24) comprenant la seconde partie (30).

9. Stent (10) selon la revendication 1, dans lequel l'alliage de molybdène/rhénium a une densité d'au moins 8 g/cm³.

10. Stent (10) selon la revendication 9, dans lequel l'alliage de molybdène/rhénium a une densité inférieure à 19 g/cm³.

11. Stent (10) selon la revendication 1, dans lequel l'alliage de molybdène/rhénium a une résistance à la traction comprise entre environ 276 MPa (40 ksi) et environ 2 068 MPa (300 ksi).

12. Stent (10) selon la revendication 1, dans lequel l'alliage de molybdène/rhénium a un module d'élasticité compris entre environ 324 054 (47 000) et environ 461 949 MPa (67 000 ksi).
